Europäisches Patentamt

European Patent Office

Office européen des brevets

(19)

(11) Veröffentlichungsnummer: **0 267 999 B1**

(12) **EUROPÄISCHE PATENTSCHRIFT**

(45) Veröffentlichungstag der Patentschrift: **21.08.91**

(51) Int. Cl.5: **A61F 5/01**

(21) Anmeldenummer: **86890324.6**

(22) Anmeldetag: **19.11.86**

(54) **Kniegelenkschiene.**

(43) Veröffentlichungstag der Anmeldung:
**25.05.88 Patentblatt 88/21**

(45) Bekanntmachung des Hinweises auf die
Patenterteilung:
**21.08.91 Patentblatt 91/34**

(84) Benannte Vertragsstaaten:
**AT BE CH DE FR GB IT LI NL SE**

(56) Entgegenhaltungen:
EP-A- 0 070 411     DE-C- 2 432 766
FR-A- 2 231 356     US-A- 2 877 033
US-A- 4 337 764     US-A- 4 361 142
US-A- 4 523 585

(73) Patentinhaber: **Sövegjarto, Harald**
**Rudigierstrasse 8a**
**A-4020 Linz(AT)**

(72) Erfinder: **Sövegjarto, Harald**
**Rudigierstrasse 8a**
**A-4020 Linz(AT)**

(74) Vertreter: **Atzwanger, Richard, Dipl.-Ing.**
**Mariahilfer Strasse 1c**
**A-1060 Wien(AT)**

# Beschreibung

Die Erfindung betrifft eine Kniegelenkschiene nach dem Oberbegriff des Anspruchs 1. Eine derartige kniegelenkschiene ist aus der US-A-4523 585 bekannt.

Es sind Kniegelenksschienen bekannt, bei welchen die beiden Schaftteile mittels eines Scharniergelenkes miteinander verbunden sind. Diese bekannten Kniegelenksschienen entsprechen jedoch deshalb nicht den an sie gestellten Anforderungen, da die Bewegung des Knies, welche durch eine kombinierte Dreh- und Gleitbewegung gebildet wird, nicht in der erforderlichen Weise nachgeahmt wird.

Aus der DE-PS 24 32 766 ist ein in der Literatur als Gelenk nach Menschik bezeichnetes Kniegelenk bekannt, durch welches die Bewegung eines Knies optimal nachgeahmt wird. Dieses bekannte Gelenk dient jedoch ausschließlich als Ersatz für ein Knie, also als Implantat. Hiefür müssen der Oberschenkelteil des Gelenkes in den Oberschenkel und der Unterschenkelteil des Gelenkes in den Unterschenkel eines Patienten implantiert werden. Für den Einsatz dieses künstlichen Kniegelenkes ist es demnach erforderlich, die Knochenteile des Knies zu entfernen und diese durch das künstliche Kniegelenk zu ersetzen. Demgegenüber bleiben die Bänder des Knies erhalten. Wie daraus verständlich ist, ist somit für den Einsatz dieses Kniegelenkes eine äußerst schwierige Operation erforderlich, welche bedingt, ein vorhandenes Knie, selbst wenn es noch beschränkt funktionsfähig ist, zu entfernen und dieses durch das künstliche Kniegelenk zu ersetzen. Dieses Implantat erfordert langandauernde Heilungsprozesse bzw. bedingt oftmals Komplikationen im Heilungsprozeß. Aufgrund dieser Tatsachen haben sich künstliche Kniegelenke nach Menschik so wenig bewährt, daß sie kaum für längere Zeit zum Einsatz kamen.

Demgegenüber liegt der gegenständlichen Erfindung die Aufgabe zugrunde, eine Kniegelenkschiene zu schaffen, die mit einem Kniegelenk ausgebildet ist, welches es ermöglicht, exakt die Bewegung des Knies auszuführen. Eine derartige Kniegelenksschiene wird am Oberschenkel und am Unterschenkel des Benützers, insbesondere eines Patienten, befestigt, wobei dann das Kniegelenk nicht anstelle des Knies tritt, sondern vielmehr zu dessen Unterstützung dient.

Der Erfindung liegt dabei die Aufgabe zugrunde, die den bekannten Kniegelenksschienen anhaftenden Nachteile, welche darin bestehen, daß sie die Bewegung des Knies nicht nachahmen, zu vermeiden. Lösung der Aufgabe ist in den kennzeichnenden Merkmalen des Anspruchs 1 angegeben.

Die Verwendung einer derartigen Kniegelenksschiene ist z.B. dann erforderlich, wenn ein nicht voll funktionsfähiges Knie entlastet werden soll, z.B. wenn nach einer Operation der Heilungsprozeß gefördert werden soll. Bisher mußten nach Kniegelenksoperationen Gipsverbände angelegt werden, wodurch jedoch den Heilungsprozeß fördernde Bewegungen des Knies verhindert wurden. Durch eine erfindungsgemäße Kniegelenksschiene kann das Knie abgestützt und hierdurch entlastet werden, ohne daß dessen Bewegung behindert wird, da durch diese Kniegelenksschiene die Abwinkelbewegung des Knies weitestgehend nachgeahmt wird.

Nach einer bevorzugten Ausführungsform sind die beiden Schaftteile im Bereich des Gelenkes mit sich in der Schwenkebene erstreckenden Verbreiterungen ausgebildet, an welchen die Lenker gelagert sind und welche für die Verschwenkung der beiden Schaftteile Anschläge bilden. Vorzugsweise ist einer der Lenker durch einen Kreissektor gebildet, der mit einem kreisförmigen Schlitz ausgebildet ist, welcher von einem in einem der beiden Schaftteile gehalterten Bolzen durchsetzt ist, wobei zur Halterung des Bolzens in unterschiedlichen Lagen mehrere Bohrungen vorgesehen sind. Weiters sind vorzugsweise zumindest zwei der einander zugeordneten Lenker an beiden Paaren mittels eines Bügels starr verbunden.

Eine erfindungsgemäße Kniegelenksschiene ist nachstehend anhand eines in der Zeichnung dargestellten Ausführungsbeispiels näher erläutert. Es zeigen:

Fig.1 eine erfindungsgemäße Kniegelenksschiene in Strecklage, in Seitenansicht, und

Fig.2 die Kniegelenksschiene nach Fig.1 in Beugelage, in Seitenansicht.

Die erfindungsgemäße Kniegelenksschiene 1 besteht aus einem Oberschenkelschaft 2 und aus einem Unterschenkelschaft 3, welche beiden Teile durch zwei Gelenke, die sich in der bestimmungsgemäßen Verwendung der Kniegelenksschiene seitlich des Knies befinden, miteinander verbunden sind. Diese Gelenke sind durch einander kreuzende Lenker 4 und 5 nach Art eines überschlagenen Gelenksvierecks gebildet. Die Lenker 4 sind im hinteren Bereich des Oberschenkelschaftes 2 mittels eines Lagers 4a und im vorderen Bereich des Unterschenkelschaftes 3 mittels eines Lagers 4b jeweils seitlich angelenkt. Demgegenüber sind die Lenker 5 im mittleren Bereich des Oberschenkelschaftes 2 mittels eines Lagers 5a und im hinteren Bereich des Unterschenkelschaftes 3 mittels eines Lagers 5b jeweils seitlich angelenkt. Durch diese einander kreuzenden Lenker 4 und 5 können der Oberschenkelschaft 2 und der Unterschenkelschaft 3 relativ zueinander eine Schwenkbewegung ausführen, welche der Roll-Gleitbewegung, die durch ein Knie ausgeführt wird, weitestgehend entspricht.

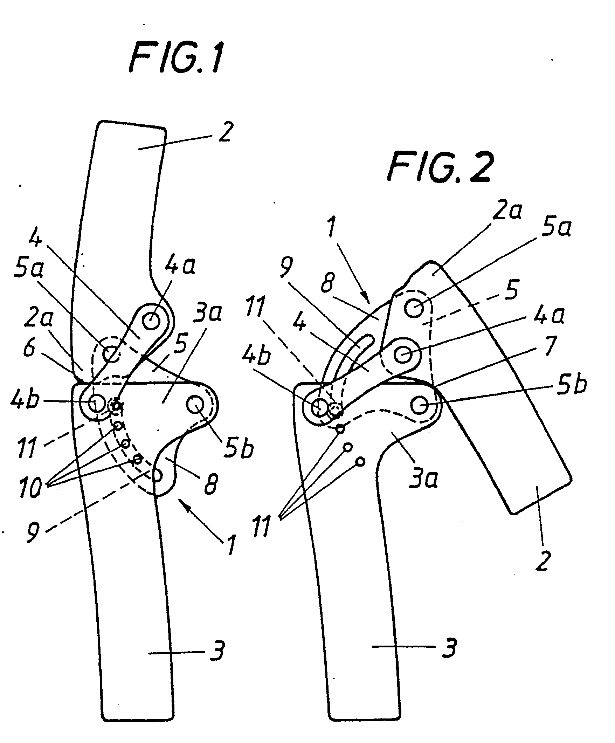

Nach einer bevorzugten Ausführungsform ist der Lenker 5 als Kreissektor 8 ausgebildet, der mit einem zum Lager 3b kreisförmig verlaufenden Schlitz 9 versehen ist. Diesem Schlitz 9 sind im oberen Bereich 3a des Unterschenkelschaftes 3 im Abstand voneinander befindliche Bohrungen 10 zugeordnet, in welche ein Zapfen 11 einsetzbar ist. Hierdurch kann das Ausmaß der Schwenkbewegung des Oberschenkelschaftes 2 gegen-über dem Unterschenkelschaft 3 begrenzt werden, wie dies aus Fig.2 der Zeichnung ersichtlich ist. In der Strecklage der Kniegelenksschiene 1 kommt der untere Bereich 2a des Oberschenkelschaftes 2 an einer Gegenfläche 6 des Unterschenkelschaftes 3 zur Anlage.

Eine anmeldungsgemäße Kniegelenksschiene 1 kommt so zum Einsatz, daß der Oberschenkelschaft 2 einerseits und der Unterschenkelschaft 3 andererseits, z.B. mittels aus steifelastischem Material gefertigten Manschetten, mit dem Oberschenkel und mit dem Unterschenkel eines Benützers, z.B. eines Patienten, verbunden werden. Bei Bewegung des Beines, also bei Verschwenkung des Unterschenkels gegenüber dem Oberschenkel wird das Knie entlastet, wodurch z.B. ein Heilungsprozeß gefördert wird. Voraussetzung für die Wirksamkeit dieser Kniegelenksschiene ist dabei, daß die die beiden Schaftteile verbindenden Gelenke Bewegungen ausführen, welche der Bewegung des Knies gleich sind.

Da für die Funktion dieser Kniegelenksschiene eine achsgerechte Führung der Lenker erforderlich ist, ist es zweckmäßig, zumindest zwei einander entsprechende Lenker mittels eines Bügels miteinander starr zu verbinden. So kann beispielsweise im Bereich des Oberschenkels ein Bügel vorgesehen sein, der die beiden Lenker 5 an der Vorderseite des Oberschenkels miteinander verbindet und kann im Bereich des Unterschenkels ein Bügel vorgesehen sein, der die beiden Lenker 4 an der Rückseite des Unterschenkels miteinander verbindet.

Da durch ein derartiges Gelenk die Drehbewegung des Knies weitestgehend nachgeahmt wird, kann durch eine erfindungsgemäße Kniegelenksschiene ein Knie wirksam entlastet werden, wodurch weitere Schädigungen eines nicht vollfunktionsfähigen Knies verhindert werden bzw. aufgrund der Entlastung ein Heilungsvorgang des Knies beschleunigt wird.

**Patentansprüche**

1. Kniegelenksschiene mit einem zur Befestigung am Oberschenkel eines Benützers, insbesondere eines Patienten, bestimmten Oberschenkelschaft (2) und einem zur Befestigung am Unterschenkel des Benützers bestimmten Unterschenkelschaft (3), welche beide Schaftteile miteinander durch zwei voneinander in einem der Breite des Knies entsprechenden Abstand angeordnete und seitlich des Knies befindliche Gelenke verbunden sind, wodurch sie gegeneinander in einer Ebene verschwenkbar sind, wobei jedes Gelenk durch einander kreuzende Lenker (4,5) gebildet ist, dadurch gekennzeichnet, daß sich in der Gebrauchslage der Schiene die Anlenkpunkte (4b, 5b) der Lenker (4,5) am Unterschenkelschaft (3) etwa in gleicher Höhe befinden und in der Strecklage der beiden Schäfte (2,3) der den vorderen Bereich des Unterschenkelschaftes (3) mit dem hinteren Bereich des Oberschenkelschaftes (2) verbindende Lenker (4) mit der die beiden Schäfte (2,3) durchsetzenden Längsachse einen Winkel von etwa 50° einschließt und daß die Abstände der Anlenkpunkte (4a, 4b bzw. 5a, 5b) der beiden Lenker (4,5) voneinander angenähert gleich groß sind und zudem dem Abstand der Anlenkpunkte (4b, 5b) der beiden Lenker (4,5) am Unterschenkelschaft (3) angenähert gleich sind.

2. Kniegelenksschiene nach Patentanspruch 1, dadurch gekennzeichnet, daß die beiden Schaftteile (2,3) im Bereich des Gelenkes mit sich in der Schwenkebene erstreckenden Verbreiterungen ausgebildet sind, an welchen die Lenker (4,5) angelenkt sind und welche für die Verschwenkung der beiden Schaftteile (2,3) Anschläge bilden.

3. Kniegelenksschiene nach einem der Ansprüche 1 und 2, dadurch gekennzeichnet, daß der eine Lenker durch einen Kreissektor (8) gebildet ist, der mit einem kreisförmigen Schlitz (9) ausgebildet ist, welcher von einem in einem der beiden Schaftteile gehalterten Bolzen (11) durchsetzt ist, wobei zur Halterung des Bolzens (11) in unterschiedlichen Lagen mehrere Bohrungen (10) vorgesehen sind.

4. Kniegelenksschiene nach einem der Ansprüche 1 bis 3, dadurch gekennzeichnet, daß zumindest zwei der einander zugeordneten Lenker (4,5) der beiden Paare miteinander mittels eines Bügels starr verbunden sind.

**Claims**

1. Knee-joint splint with an upper leg shaft (2) intended for attachment to the upper leg of a user, in particular a patient, and with a lower leg shaft (3) intended for attachment to the lower leg of the user, which two shaft parts are connected to each other by means of two hinges arranged at a distance from each other corresponding to the width of the knee and situated to the sides of the knee, as a result of which they can be pivoted relative to one another in one plane, each hinge being formed by links (4, 5) crossing one another, characterised in that in the use position of the splint the articulation points (4b, 5b) of the links (4, 5) on the lower leg shaft (3) are approximately at the same level, and in the extension position of the two shafts (2, 3) the link (4) connecting the front area of the lower leg shaft (3) with the rear area of the upper leg shaft (2) encloses with the longitudinal axis passing through the two shafts (2, 3) an angle of approximately 50°, and in that the distances of the articulation points (4a, 4b and 5a, 5b) of the two links (4, 5) from each other are approximately of the same size, and in addition are approximately equal to the distance between the articulation points (4b, 5b) of the two links (4, 5) on the lower leg shaft (3).

2. Knee-joint splint according to Patent Claim 1, characterised in that the two shaft parts (2, 3) are designed in the area of the hinge with widened parts extending in the pivot plane, on which widened parts the links (4, 5) are articulated, and which form stops for the pivoting of the two shaft parts (2, 3).

3. Knee-joint splint according to one of Claims 1 and 2, characterised in that the one link is formed by a circle sector (8) which is designed with a circular slot (9), through which there passes a pin (11) held in one of the two shaft parts, a plurality of bores (10) being provided for holding the pin (11) in different positions.

4. Knee-joint splint according to one of Claims 1 to 3, characterised in that at least two of the mutually associated links (4, 5) of both pairs are connected rigidly to one another by means of a strap.

**Revendications**

1. Attelle pour articulation du genou comportant une tige de fémur (2) destinée à être fixée sur le fémur d'un utilisateur, notamment d'un patient, et une tige de jambe (3) destinée à être fixée sur la jambe de l'utilisateur, les deux parties de tige étant assemblées entre elles par deux articulations, placées espacées l'une de l'autre d'une distance correspondant à la largeur du genou et se trouvant sur le côté du genou, ce qui les rend orientables l'une par rapport à l'autre dans un plan, chaque articulation étant formée par des bras oscillants (4, 5) se croisant l'un l'autre, caractérisée en ce qu'en position d'utilisation de l'attelle, les points d'articulation (4b, 5b) des bras oscillants (4, 5) sur la tige de jambe (3), se trouvent à peu près à même hauteur et en position tendue des deux tiges (2, 3), le bras oscillant (4) reliant la zone avant de la tige de jambe (3) à la zone arrière de la tige de fémur (2), forme un angle de 50° environ avec l'axe longitudinal traversant les deux tiges (2, 3) et en ce que les distances séparant les points d'articulation (4a, 4b ou 5a, 5b) des deux bras oscillants (4, 5), sont à peu près les mêmes et en outre à peu près égales à la distance séparant les points d'articulation (4b, 5b) des deux bras oscillants (4, 5) sur la tige de jambe (3).

2. Attelle pour articulation du genou selon la revendication 1, caractérisée en ce que les deux parties de tige (2, 3) présentent, dans la région de l'articulation, des élargissements s'étendant dans le plan de pivotement, auxquels s'articulent les bras oscillants (4, 5) et qui forment des butées pour le pivotement des deux parties de tige (2, 3).

3. Attelle pour articulation du genou selon l'une des revendications 1 ou 2, caractérisée en ce qu'un bras oscillant est formé par un secteur de cercle (8) qui présente une fente (9) circulaire laquelle est traversée par un goujon (11) maintenu dans l'une des deux parties de tige, plusieurs trous (10) étant prévus pour maintenir le goujon (11) dans différentes positions.

4. Attelle pour articulation du genou selon l'une des revendications 1 à 3, caractérisée en ce que deux au moins des bras oscillants (4, 5) associés l'un à l'autre des deux paires, sont assemblés rigidement entre eux au moyen d'un étrier.

# FIG.1

# FIG.2